# EUROPEAN PATENT SPECIFICATION

(11) **EP 1 596 820 B1**
(45) Date of publication and mention of the grant of the patent: **05.11.2008**
(21) Application number: 04715264.0
(22) Date of filing: 27.02.2004
(51) Int. Cl.: A61Q 19/00, A61K 8/18

(54) **AMBIPHILIC POROUS MATRIX**
AMBIPHILE PORÖSE MATRIX
MATRICE POREUSE AMBIPHILE

(30) Priority: 27.02.2003 EP 03251212
(43) Date of publication of application: 23.11.2005
(73) Proprietor: PHARES PHARMACEUTICAL RESEARCH N.V., Curaçao (AN)
(72) Inventor: LEIGH, Mathew, Louis, Steven, CH-4052 Basel (CH); LEIGH, Steve, 1000 CX Amsterdam (NL); KUNG, Elsa, CH-4054 Basel (CH); VAN HOOGEVEST, Peter, CH-4416 Bubendorf (CH)
(74) Representative: Hasler, Erich
(86) International application number: PCT/EP2004/001985
(87) International publication number: WO 2004/075872

(56) References cited:
- WO-A-98/48774
- US-A- 5 143 940
- US-A1- 2001 009 671
- US-A1- 2002 031 534
- US-A1- 2003 235 597

## Description

### FIELD OF THE INVENTION

This invention relates to skin care compositions which adsorb or absorb sebum and transfer beneficial oils and hydrating components from a reservoir provided by a porous ambiphilic matrix.

### BACKGROUND TO THE INVENTION

Sebaceous glands are located in the hair follicles and throughout the skin but not the soles of the feet and palms of the hand. Together with sweat glands they are a necessary part of normal skin physiology, as the sebum helps to maintain hair and skin lubricity and prevents excess loss of moisture. Sebum is made up of fatty acids, esters, glycerides and other endogenous lipids. Along with natural moisturizers in the epidermis, it helps to keep the skin soft and hydrated. Production of natural oils is influenced by hormonal and environmental factors and exacerbated by elevated temperatures. In teenagers and some adults, there may be over production and this can result in oily or greasy skin and scalp (hair).

The problem is particularly noticeable around exposed regions such as the forehead and the triangular area bordering the nose. Freshly secreted sebum has some antibacterial properties and is not harmful. However where there is excess secretion and it is not washed away or removed, the sebum can combine with cell debris and pollutants to form waxy plugs or comedones which block pores and encourage bacterial colonization. Comedones are implicated in some forms of acne.

Document US2001/009671 describes a cosmetic composition comprising a panthenol oil regulating agent, a particulate oil-absorbing polymer and a cosmetically acceptable carrier. The polymer oil-absorbing agent is in the form of a powder, the powder being a combined system of particles and the composition comprises hydrophilic components, e.g. thickening agents such as cetyl hydroxyethyl cellulose, surfactants, lipophilic components, e.g. emollients such as dimethicone and biologically active compounds, e.g. sunscreens.

Document WO98/48774 refers to a cosmetic composition for topical application comprising agglomerated silica and/or talcum and other cosmetic ingredients, e.g. triglycerides, carboxy vinyl polymer, surfactants and UV light absorbents.

Document US2002/031534 discloses a fluid cosmetic composition comprising a sebum-absorbing agglomerated silica and/or talcum, a lipophilic component, a hydrophilic component and a surfactant agent.

Other than cleansing lotions, facial washes and shampoos, various skin care products are widely used to address the aesthetic problems associated with oily skin and greasy hair. However there is a need for more user friendly skin care products that adsorb/absorb and mop up excess oils through sebum regulation without dehydrating the epidermis or damaging hair.

Skin care compositions that are used for covering up oily skin are traditional natural adsorbents such as silicones, talcum powder, and clays such as bentonites Broadly, there are three types of application for removing or mopping up oils on oily skin. In one approach the compositions are applied as such from compressed powder cakes as fine powders to adsorb oily patches. The problem with fine powders is that the natural unmodified particles may not have sufficiently extensive surfaces. Small particles block pores and prevent the skin from transpiring. The second approach is more of a treatment where a suspension or paste is applied as a face mask and allowed to dry before removal. Although more effective, the drawback with this method is that the mask has to be left on and is not convenient for routine use. The treatment also dries and deoils the skin by absorbing and removing natural lubricants and moisture. More usually, the powders and/or anti-sebum components are incorporated into tonics, aqueous creams and gel like compositions which may contain alcohol to give the skin a matte, non glossy appearance. However, the compositions as a whole may simply dehydrate the skin and mop up oily secretions briefly after application. Environmental pollutants such as fumes from car exhausts can also add to the challenge in dealing with oily skin.

Although the invention relates to the treatment of oily skin, it may equally apply to a method of reducing trans epidermal water loss(TEWL) which is a cause of dry skin. TEWL may be due to removal of natural oils and exacerbated by a dry environment. The skin needs to be kept naturally lubricated and moist.

### SUMMARY OF THE INVENTION

The invention is in the area of 'porous oil adsorber' and 'porous ambiphilic matrix' for sebum regulation and/or skin hydration. The compositions comprise synthesised porous agglomerates and have longer acting capacity to absorb oil and transfer lipophilic components as well as provide a moist, compatible micro environment to enable hydration or rehydration in skin care.

The invention describes skin care compositions as described in claim 1 to;
i) mop up and remove sebum and allow the transfer of beneficial oils to the skin,
ii) provide hydrating components from a reservoir comprising synthetic porous particles, lipophilic and hydrophilic ingredients.

The compositions may be used as such in particulate form or added as a powder composition concentrate to a carrier system or external medium which may be hydrophilic, lipophilic, particulate, or anhydrous. Preferably the powder concentrate has a water content below 20 wt %. The powder concentrate may also be used to prepare powder or paste like compositions using diluents, fillers or particulate bulking agent/s depending on the type of cosmetic application.

In one aspect, the invention describes a powder composition, semi-solid or liquid concentrate that may be included in skin care compositions to absorb sebum and replenish the skin with beneficial oils as well as to transfer hydrating ingredients from an ambiphilic porous matrix into the skin.

Therefore, the present invention relates to:
An ambiphilic solid particle composition for use in dermal (topical) composition, which comprises
   a) water insoluble porous particles of organic and/or inorganic compounds with specific surface area greater than 40 m²/g,
   b) at least one lipophilic component,
   c) at least one hydrophilic component,
   d) at least one physiologically acceptable surfactant, and, optionally,
   e) a biologically active compound and other excipients.

The invention further relates to a dermal or topical composition wherein the above-mentioned composition is dispersed, which essentially consists of 1 wt % to 50 wt % of the synthetic porous agglomerates of water-insoluble solid particle of organic and/or inorganic compounds with specific surface area greater than 40m²/g, and, optionally , at least one biologically active compound and, optionally further pharmaceutically acceptable ingredients. The dermal or topical composition may be a lotion, gel, cream, stick, powder, compressed cake, or paste like skin care composition prepared by homogeneously mixing and dispersing 1 wt % to 10 wt % of the powder composition concentrate in a suitable base carrier.

The invention further describes a method to absorb sebum and/or hydrate skin using said powder, semi solid or liquid concentrate with a water content preferably less than about 20 wt %, uniformly dispersed either in an anhydrous base or carrier, or in an external water phase. Where the external medium is water the aqueous dispersion equilibrates to a hydrated porous ambiphilic matrix and forms a reservoir on the skin for the transfer of lubricating and hydrating component/s by a process of diffusion. Alternatively when an anhydrous external phase other than water is used, the compositions comprising dispersed ambiphilic porous particles in a matrix may be used to facilitate transfer of both lipophilic and hydrating components contained therein to the epidermis thereby reducing TEWL whilst maintaining skin lubricity and hydration.

### DETAILED DESCRIPTION OF THE INVENTION

'oils' in this specification include not only sebum secreted by sebaceous glands but also olioginous substances.
'anti-sebum' components are biologically active compounds which influence sebum secretion.
'hydrophilic' compounds are non volatile and dissolve easily in water. The definition includes hydrophilic polymers, hydro colloids and natural gums. They have the capacity to bind onto water molecules (hydro active) and aid skin rehydration and reduce trans epidermal water loss (TEWL) by maintaining a moist, humid micro-environment over the skin.
"lipophilic" describes a liquid or semi solid compound that has the properties of an oil and is miscible or compatible with sebum.
'ambiphilic' or 'amphiphilic' describes a composition which has both hydrophilic and lipophilic properties, i.e. it has affinity for water as well as oil.
'adsorb' or adsorption is a surface phenomenon whereby water or oil molecules are attached to the surfaces of single insoluble particles. In this specification the single particles form highly porous agglomerates which 'absorb' oils.
'absorb' or absorption refers to the immobilization or 'mopping up' of oils (likened to a sponge) by the multi-component skin care compositions comprising porous agglomerates described in this specification. The terms 'adsorb' and 'absorb' are used interchangeably to describe this effect.
'skin' in this specification includes scalp and all external body surfaces.
'skin-care' refers to compositions, products and external applications that confer beneficial physiological, functional, aesthetic and cosmetic properties and are perceived to improve skin condition. The definition includes but not limited to aqueous, aqueous-alcohol, non aqueous, lipophilic and dispersed systems such as suspensions, slurries, pastes, gels, lotions, emulsions, sticks, powder blends, compressed powders, and such like compositions.
'dispersion' includes all types of multi component compositions including but not limited to lotions, gels, creams, ointments, sticks, powders and paste-like compositions comprising porous particles uniformly distributed or homogeneously suspended in water or non aqueous, lipophilic, waxy and particulate medium.
"powder concentrate" describes powder blends that may be free flowing compositions or suspensions of synthetic porous agglomerates comprising solid particles of organic and/or inorganic compounds with surface areas of at least 40m²/gm {Method: Gas adsorption(BET)} that are used in skin care compositions for adsorbing sebum and delivering lipophilic and hydrophilic ingredients to the skin.
"waster insoluble" refers to compounds that have a solubility of less than 1:10 in water at pH 7.

The invention describes novel ambiphilic compositions comprising synthetic inorganic or organic water insoluble porous agglomerates or mixtures of different types of synthetic agglomerates characterized by surface areas of at least 40m²/g up to 900m²/g or more comprising agglomerates of individual or single particles within the size range between 0.05µm to 0.1µm, with the capacity to absorb at least an equal volume by weight of a liquid. The porous agglomerates are mixed with functional ingredients which include lipophilic, hydrophilic and surface active components to prepare an ambiphilic powder concentrate. Preferably, the compositions may further include other biologically or pharmacologically active compounds and stabilisers such as anti microbials, antioxidants and pH buffers. The powder concentrate may be used as such or added to all types of carrier systems used as skin care compositions and applications.

The invention further describes a method for sebum regulation by absorbing or mopping up oil on skin surfaces by forming an ambiphilic matrix type reservoir that absorbs sebum and transfers beneficial oils and hydrating components to the skin under a humid micro environment. The effects last upwards of 4 hrs after application. Optionally, the reservoir may contain biologically active components with or without additional anti- sebum effects.

According to one embodiment, the invention is an ambiphilic particulate, composition. The powder mixture may be used as a concentrate to prepare dispersed aqueous dermal compositions such as slurries or pastes, hydrophilic creams, tonics or gels for cosmetic and other topical applications. The slurries or paste like compositions may be milled to obtain homogeneity and blended with particulate carriers or bulking agents such as talc, kaolin, etc. The ambiphilic powder mixture is also suitable for use in non-aqueous, wax like compositions such as lip sticks and water-in-oil cosmetic compositions to provide a porous hydrated matrix and micro reservoir for extended transfer of both lipophilic and hydrophilic components and optionally biologically active compounds. Furthermore, the invention may be used in hair care products, such as shampoos and conditioners to absorb excess oil from scalp and hair and replenish with conditioning components and moisturizers.

The ambiphilic powder composition concentrate comprises 10 wt % to 95 wt % of water insoluble synthetic porous agglomerates of organic and/ or inorganic compounds with surface area greater than 40 m²/g,
a) 1 wt % to 50 wt % of at least one lipophilic component compatible with sebum,
b) 0.5 wt % to 50 wt % of at least one hydrophilic component,
c) 0.1 wt % to 25 wt % of at least one pharmaceutically acceptable surfactant and
d) 1 wt % to 20 wt % water or bound moisture and, optionally, 1 wt % to 10 wt % of one or more biologically active compound, stabilizers, preservatives.

According to a preferred embodiment the ambiphilic powder composition concentrate comprises,
a) 25 wt % to 75 wt %, of at least one type of water insoluble porous agglomerates of organic and/or inorganic compounds with surface area greater than 40 m ²/gm,
b) 5 wt % to 25 wt % of at least one lipophilic component miscible with sebum,
c) 1 wt% to 25 wt %of at least one hydrophilic component,
d) 0.5 wt % to 10 wt % of at least one physiologically acceptable surfactant,
e) less than 10 wt% water or bound moisture,
f) Optionally 1 wt % to 10 wt % of one or more biologically active compound, stabilizers, preservatives.

In a preferred embodiment the powder concentrate is incorporated in an aqueous suspension which is applied as such to the skin for absorbing, covering, mopping up oils and transfer hydro active, hydrophilic components to the skin to improve water binding capacity and reduce trans epidermal water loss. The aqueous composition dries and equilibrates to a matte, non lustrous ambiphilic porous matrix which forms a reservoir on the skin surface.

Preferably, the aqueous dispersion is prepared by homogeneously suspending and mixing the said powder mixture concentrate in an aqueous medium or base composition comprising additional excipients or bulking agents conventionally used to prepare lotions, gels, creams or paste-like compositions. Although up to 50 wt % of the powder concentrate may be used, normally between 1 wt % to 10 wt % of the said ambiphilic powder mixture is all that is required in the aqueous dispersion. Alternatively, an aqueous suspension may be prepared by combining and mixing together all the separate, individual components. In the event, the total amount of lipophilic and hydrophilic ingredients in the final skin care composition may exceed the amounts used in preparing a porous ambiphilic powder concentrate. The skin care composition may be a lotion, tonic, gel, emulsion or paste-like suspension.

Accordingly, the invention further describes a lotion, tonic, gel, emulsion, cream or paste-like aqueous suspension comprising;
a) 0.5 wt % to 50 wt % of said powder concentrate comprising essentially porous particles with surface area greater than 40m²/g, preferably 1 wt % to 10 wt %, most preferably 2 wt % to 5 wt %,
b) 0.25 wt % to 75 wt % of lipophilic components, preferably 1 wt % to 10 wt %,
c) 0.1 wt % to 75 wt % of at least one hydrophilic components, preferably 0.5 wt % to 10 wt %,
d) 0.1 wt % to 10 wt % of surfactants or emulsifiers, preferably 0.5 wt % to 2.5 wt %,
e) 10 wt % to 99 wt % by weight of water, or hydrophilic or lipophilic or particulate carrier, preferably 10 wt % to 90 wt%
f) Optionally 0.1 wt % to 10 wt % of a biologically active compound, stabilizers, preservatives.

The suspension is applied for sebum regulation and on drying at equilibrium, it provides a biologically compatible, hydrated and moist ambiphilic matrix reservoir that
a) absorbs sebum/oils from the skin/scalp in situ, b) transfers biologically beneficial lipophilic components or natural oils to the skin by means of equilibrium diffusion, c) maintains a moist and humid micro environment for skin hydration or rehydration. Therefore the skin is neither defatted nor dehydrated. The adsorbed/absorbed oil/sebum is held by the porous particle agglomerates and the external ambiphilic matrix which may further contain a lipophilic or hydrophilic polymer or hydrocolloid. The particles are embedded in the polymeric carrier which prevents them from clogging up pores.

Although aqueous based systems may be used as preferred carriers in skin care and foundation creams for preventing oily skin and provide skin hydration, the ambiphilic powder concentrate is just as suitable for powder application in compressed cakes, by blending uniformly with fillers and diluents. Examples of other types of non aqueous skin care compositions are lip sticks, lip balms, eye lash mascaras, eye shadows, and compressed powders or blushers for colour cosmetics.

The functional components which may be used in the ambiphilic powder concentrate and resultant skin care compositions include but are not limited to one or more of the following ingredients:

### a) ABSORBENT

Components that may be used as absorbents for sebum regulation are water insoluble synthetic porous agglomerates comprising solid particles with specific surface area within the range from 40 m²/g to 900 m²/g, or more, preferably between 75 m² / g to 300 m²/g, more preferably, between 150 m²/g to 500 m²/g. The mean particle diameter of the agglomerates lie between 1 µm-10 µm preferably 1 µm - 5 µm. Each single, individual particle is between 0.01 µm to 0.1 µm, preferably 0.01 µm - 0.05 µm, mean diameter. The porous agglomerates have the capacity to absorb between 1- 5 times or more its weight of a liquid.

According to a preferred embodiment, the invention comprises at least one synthetic porous material selected from the group consisting of, for example, amorphous aluminometasilicate, calcium silicate, silica, including porous silicon dioxide and silicic acid, dibasic anhydrous calcium phosphate and polystyrene beads/micro-sponges.

Preferred absorbents which are employed in this invention include inorganic solids, e.g. calcium hydrogen phosphate, which corresponds to the general formula CaHPO₄•mH₂O (m: 0 ≤ m ≤ 2) commercially available as Fujicalin®; magnesium aluminometasilicate, which corresponds to the general formula Al₂O₃•MgO•2SiO₂•n H₂O (n: 0 ≤ n ≤ 2), commercially available as Neusilin®; calcium silicate, e.g. Ca2SiO4 or Ca3SiO, and dibasic anhydrous calcium phosphate.

In a preferred embodiment the invention comprises as component a) a porous material selected from the group consisting of amorphous Al₂O₃•MgO•₂SiO₂•nH₂O, CaHPO₄•mH₂O, CaSiO₄, Ca₃SiO₅ and dibasic anhydrous calcium phosphate.

Suitable FUJICALIN products are SG and S and characterised by a mean pore size of 7,0 x 10⁻¹⁰ m, a mean particle size of about 2 µm -10 µm, a specific volume of about 1.5 ml/ g or more, a BET specific surface area of 20 m²/g to 60 m²/g, and an oil and water absorption capacity of about 0.7 ml/g.

Suitable NEUSILIN products are Grades S1, SG1, UFL2, US2, FH2, FL1, FL2, S2, SG2,NFL2N and NS2N. Particularly preferred grades are S1, SG1 US2 and UFL2. These materials, which are amorphous, typically have a specific area of about 100 m²/g to about 300 m²/g, an oil absorption capacity of about 1.3 ml/g to about 3.4 ml/g, a mean particle size of about 1 µm to about 2 µm and a specific volume of about 2.1 ml/g to about 12 ml/g.

Other suitable materials are precipitated amorphous calcium silicate, e.g. Zeopharm® 600 with a surface area of 300 m²/g and oil absorption capacity of at least 450 ml/100g, Hubersorb® 250 NF with oil absorbing capacity of 250 - 300 ml/100 g, and Zeopharm® 80 (precipitated amorphous silica) with surface area of 140 m²/g and oil absorption capacity of 185 - 215 ml/100 g. Alternative types of organic or inorganic porous materials may be used, as long as they have no deleterious effect on the active compound and present internal surface areas which are comparable. The invention also allows for compositions comprising combinations of different types of porous particles and agglomerates that may have superior oil absorption properties compared to a single type.

It should be clearly understood that although the examples illustrated are mostly porous agglomerates of organic and/or inorganic compounds, water insoluble porous agglomerates prepared from organic materials such as polystyrene with surface areas not less than 40 m²/g may be used in partial or complete replacement

### b) LIPOPHILIC COMPONENT

The lipophilic component may be a functional oil which is compatible with and exchanges for sebum. However, minor amounts of lipophilic polymers such as low viscosity grades of ethyl cellulose and natural gums such as Dammar gum with solubilities of less than 10 wt%, that either swell or dissolve in oils may also be regarded as lipophilic component and lie within the scope of this invention. Preferably the polymer or lipophilic ingredient is complexed with the absorbent to render the particles lipophilic without saturating the absorption surfaces. This assists in attracting lipophilic materials such as sebum into the porous particles aided by the ambiphilic matrix. Most lipophilic or olioginous components used in cosmetic products for functional and emollient properties are suitable. Preferred oils are the mono, di and triglycerides of fatty acids particularly those from natural sources and fatty acid esters. The lipophilic component may be one or more biologically active compound. Preferred lipophilic component such as black cumin seed oil has antimicrobial properties, whilst pumpkin seed oil has anti sebum properties. Other lipophilic components which have therapeutic properties are e.g. wheat germ oil and the tocopherols. Volatile oils, some unsaturated and highly unsaturated oils with two or more double bonds may have additional anti microbial or anti inflammatory properties. Preferred oils used typically as lipophilic component are volatile silicones e.g cyclo penta siloxane, dimethicones and hydrogenated poly isobutane.

### c) HYDROPHILIC COMPONENT

These include hygroscopic and water soluble compounds that can bind on to water e.g. polyols such as glycerol, propylene glycol, hexalene glycol and sugars e.g. mannitol and sorbitol, etc. They may also be organic compounds such as hyaluronic acid, urea and betaine. Hydro-active compounds reduce trans epidermal water loss which makes the skin dry. The hydrophilic component may also be a hydrophilic polymer or a gelling agent preferably with film forming properties. Examples of hydrophilic polymers include but are not limited to the water dispersible cellulose esters and ethers, natural gums and hydrocolloids such as gelatine, collagen, proteins, amino acids, peptides and polypeptides, carbomers, carboxy vinyl co polymers and sodium acrylates. Dammar gum and low viscosity grades of ethyl cellulose, such as N7, N4 or N3 (Dow Chemical or Hercules Inc), or equivalent with limited water solubility are also suitable either as lipophilic or hydrophilic component.

### d) SURFACTANT

Surfactants are used mainly to facilitate uniform distribution of powders and stabilise dispersed phase systems. Preferred surfactants to facilitate powder dispersion are preferably amphipathic e.g. lecithins, phospholipids. Some cationic and amphoteric compounds are both surfactants and antimicrobials and may be hydrophilic. Ambiphilic surfactants are non ionic, polyethylene fatty acids and esters with HLB values typically between 2 to 18. Surface active agents used as emulsifiers to prepare and stabilise emulsion systems may be used as wetting agents to facilitate powder dispersion.
Amphoterics such as, e.g., Phosphates e.g. Phospholipids, Amine derivatives e.g. N-Alkyl amino acids, Alkylamido alkylamines.
Anionics such as, e.g., Carboxylic acids e.g. soaps, Carboxylic acid esters e.g. Lactylates, PEG alkyl carboxylates, Sulphuric acid esters e.g. Alkyl sulfates, Sulfated monoglycerides, Sulfonic acids e.g. Sulfosuccinate esters, Acyl isethionates, Amino acid amides e.g. Sarcosinates, Acylated peptides.
Cationics such as, e.g., Amines e.g. PEG Alkyl amine, Quaternaries e.g. Tetraalkyl ammonium salts.
Non-ionics such as, e.g., Alcohols e.g. fatty alcohols, Ethers e.g. Alkoxylated fatty alcohols, PEG-Phenol Ethers, Esters e.g. Alkoxylated fatty acids, Acyl sorbitans, and PEG-Derivatives, Acyl glycerides, Amides e.g. PEG Alkyl amides, Polymers e.g. PEG/PPG Block polymers, PEG silicones Derivatives, Alkyl-substituted polyvinyl polymers.

### e) PRESERVATIVES

Acids such as, e.g., Benzoic, Sorbic, Dehydroacetic.
Parabens such as, e.g., Methylparaben, Propylparaben, Ethylparaben, Butylparaben.
Quaternary Ammonium compounds such as, e.g., Benzalkonium chloride, Cetylpyridinium chloride, Benzethonium chloride.
Formaldehyde donors such as, e.g., 2-bromo-2-nitropropane-1,3-diol (BNPD), Glutaraldehyde, Quatenium 15, Imidazolidinyl urea, Diazolidinyl urea, DMDM Hydantoin.
Alcohols such as, e.g., Ethyl alcohol, Benzyl alcohol, 2,4-dichloro benzyl alcohol.
Multifunctional ingredients with antimicrobial properties such as, e.g., Caprylyl Glycol, Glyceryl Caprate, Glyceryl Caprylate, Polyglyceryl-2 Caprate, Sodium Lauroyl Lactylate.

### f) ANTIOXIDANTS:

Phenolic antioxidants e.g. Tocopherol (and derivatives), BHA, BHT, Hydroxyanisole, Rosmarinic acid etc, Carotenoids, Flavonoids -Flavanol e.g. Epicatechin, Catechin etc, Flavonol e.g. Diosmin, Kaempferol etc, Flavone e.g. Apigenin, Flavanone e.g. Naringin, Hesperidine, Anthocyanidin e.g. Malvidin, Cyanidin, Isoflavone e.g. Genistein, Phenolic acids - Hydroxylated benzoic acid, phenylacetic acids, phenylacrylic acids, Gallic acid etc, Miscellaneous - Ascorbic acid, Ascorbyl palmitate, Carotenoids, Thioglycolic acid (and derivatives) etc.

### g) BIOLOGICALLY ACTIVE COMPOUNDS

Sunscreens such as, e.g. Aminobenzoic acid, Avobenzone, Cinoxate, Dioxybenzone, Homosalate, Meradimate (Menthyl anthranilate), Octocrylene (2-ethylhexyl 2-cyano-3,3-diphenylacrylate), Octinoxate (Octyl methoxycinnamate), Octisalate (Octyl salicylate), Oxybenzone, Padimate O, Ensulizole (Phenylbenzimidazole sulfonic acid), Sulisobenzone, Titanium dioxide, Trolamine Salicylate (Triethanolamine salicylate), Zinc oxide.

The invention describes a method and composition thereof comprising (i) a porous ambiphilic matrix which absorbs excess sebum, (ii) lipophilic component/s which transfers beneficial oils and keep skin lubricated (iii) hydrophilic component/s which provides a moist micro environment and allows the skin to breathe. The invention may also be used as an improved moisturiser by reducing TEWL and allowing skin hydration or rehydration. It may be used in lip sticks, lip balms, skin and hair care products, sun screens, foundation and make up creams, etc to maintain skin hydration and/ or sebum regulation.

In vivo studies where carried out on 10 human volunteers where a composition comprising 2 wt% of the ambiphilic powder concentrate was prepared according to Example 1 and dispersed in 0.4% Ultrex® 10 and water up to 100%. The composition was tested against a control which did not contain the powder concentrate. The two compositions were applied bilaterally to the forehead. Sebum measurements at time 0, 1 hr and 4 hrs were carried out with a Sebumeter®.

After 1 hr the decrease in sebum on the treated side was 40% less than the control. After 4 hrs the rate of sebum secretion was still 7% less than the control side. The invention has the capacity to mop up sebum for more than 4 hrs after initial application.

The following example set out the typical range of preferred components in the ambiphilic powder concentrate to illustrate the invention.

### Example 1

| a) Absorbent | |
|---|---|
| ¹Neusili UFL2 | 40% - 75% wt/wt |

| b) Lipophilic component | |
|---|---|
| ²Black Cumin seed oil | 5% - 25% wt/wt |

| c) Hydrophilic component | |
|---|---|
| Hydroxyethyl cellulose | 5% - 20% wt/wt |

| d) Surfactant | |
|---|---|
| ³Phospholipid mixture | 1% -5 % wt/wt |

| | |
|---|---|
| ¹Fuji Chemical Industry ²An alternative oil or a lipophilic compound e.g. pumpkin seed oil, wheat germ oil, Vit A esters may be used in partial or complete replacement. ³ In place of phospholipids such as lecithin, ionic or non ionic surfactant/s may be used. | |

The phospholipid mixture is dispersed in the oil and adsorbed on to the Neusilin powder. Porous agglomerates consisting of other mineral oxides or mixtures thereof with surface areas within the range of 40 m²/g to 900 m²/g and similar absorptive capacity may be used in place of Neusilin UFL2 in this example. The powder complex is sifted or mixed under high shear. The hydroxyethyl cellulose is blended into the powder complex. Alternatively the hydroxyethyl cellulose may be added to the phospholipid mixture and oil prior to being adsorbed onto the Neusilin powder, and the final powder sifted or mixed under high shear. The powder composition contains less than 10 wt % of water. Optionally the powder mixture may be milled to reduce particle size. The powder mixture before or after milling, may be used in preparing a lotion, gel, cream or paste like composition. It may also be used in a base carrier for application to lips and lip sticks.

### Example 2

| | |
|---|---|
| Powder complex of Example 1 | 1 wt % to 10 wt % |
| Ultrex 10 | 0.5 wt % |
| Preservatives, stabilisers, etc | 0.1 wt % to 2wt % |
| Excipients, perfumes, etc | 1 wt % to 30 wt % |
| Demineralised water ad | to 100 wt % |

This is an example of a typical skin care composition for adsorbing/absorbing oils and hydrating skin.

The powder mixture is hydrated with about 1 to 8 times its weight of water to prepare a homogeneous and smooth slurry. The carbomer, Ultrex 10 is dispersed in the solution comprising the remainder of the water and paraben. Optionally the water is heated to about 50°C. This is added to the slurry. The pH of the suspension is adjusted to 5.5 to convert into a homogeneous gel. Preservatives such as antimicrobials, stabilisers such as buffers and antioxidants may be included in the composition in addition to any other excipients such as additional surfactants, oils and humectants. The composition is filled and packed into suitable containers such as tubes and jars.

Other types of skin care compositions suitable for use with the invention are:

### Example 3

General powder bases e.g. foundation pressed powders, foundation loose-powders, compressed powder blushers and eye shadows.

| | |
|---|---|
| *Ambiphilic porous powder concentrate from Ex 1 | 1% -10% (parts by weight) |
| Filler e.g. Talc, Mica, Sericite | 30% - 80% |
| Solid (dry) binder. Other materials | 3% -10% |
| that aid compaction, powdered polyethylene , powdered waxes, kaolin etc. | |
| Metallic soaps of fatty acids e.g.zinc stearate. | |
| Liquid (wet) binder e.g. synthetic hydrocarbons, esters and silicones | 0% - 10% |
| Pigments (&) Pearl e.g. Titanium dioxide, yellow iron oxide, Timica pearl white etc | 3 % - 50% % |
| Perfume | 0.1% - 0.5 % |
| Preservative | 0.1% - 0.5% |

| | |
|---|---|
| *The powders are blended and mixed together uniformly with the ambiphilic porous powder concentrate. In place of or in addition to the polymer, a polyol or combinations thereof may be used as hydrophilic, hydroactive ingredient. | |

### Example 4,5,6

### W/O,O/W and W/Silicone foundations

| | |
|---|---|
| Ambiphilic porous powder concentrate from Ex 1. | 1% - 100% (by weight) |
| Pigments and Filler e.g.Titanium dioxide, Talc etc | 5% - 20% |
| Dispersants e.g. Anionic and non-ionic surfactants, fatty alcohols, glyceryl esters, fatty acids, emollient oils, low viscosity esters and volatile silicone oils. | 5% - 50% |
| Suspension aids e.g magnesium. aluminium silicate, cellulose gum and xanthan gum. | 0.1% -10% |
| External phase e.g. water, humectants, oils, silicones. | 25% - 65% |
| Preservatives | 0.1% - 1% |

The ambiphilic powder concentrate is dispersed in the hydrophilic phase and added to the lipophilic ingredients containing the surfactants. The suspension is mixed homogeneously.

### Example 7,8,9

### Wax-based sticks i.e. lipsticks, wax-based blushers and eye shadows

| | |
|---|---|
| *Ambiphilic porous powder concentrate from Ex.1 | 1% - 10% (by weight) |
| Hydrophilic active e.g. Hyaluronic acid solution, water, glycerol. | 0% - 5% |
| Staining dye (bromoacids) | 0% - 0.5% |
| Insoluble pigments | 5% - 15% |
| Titanium dioxide | 0% - 7% |
| Pearlaceous pigment | 0% - 25% |
| Lipid base materials e.g. Carnuba wax, Candelilla wax, Microcrystalline waxes, amorphous hydrocarbon waxes, beeswax, cocoa butter, hydrogenated vegetable oils, lanolin and its dertivatives, petroleum jelly, polyethylene and organically modified silicones. | 5% - 20% |
| Bromoacid solvents and oils e.g. liquid fatty alcohols, dipropylene glycol, high polar esters, castor oil, volatile silicone oils etc | 10% - 80% |
| Wetting agents e.g. low HLB surfactants, lecithin | 0.01% - 3% |
| Antioxidants | 0.01% - 0.5% |
| Antimicrobials | 0.1% - 0.5% |
| Perfumes | 0.1% - 0.5% |

The polymer is omitted from the powder concentrate. In its place hydro active, hydrophilic ingredients comprising hyaluronic acid, water and glycerol are added to the lipophilic skin care compositions.

### Examples 10,11,12

### Mascaras: Anhydrous, W/O and O/W

| | |
|---|---|
| Ambiphilic porous powder concentrate from Ex1. | 1% - 10% (by weight) |
| Hydrophilic active e.g. Hyaluronic acid solution, glycerol | 0% - 5% |
| Waxes, solvents and oils e.g. candelillia wax, hydroxylated lanolin, propylene carbonate, C₁₀₋₁₃ isoparaffin | 20% - 50% |
| Water, deionised | 0% - 50% |
| Gellants and suspending agents e.g. Zinc stearate, quaternitun-18 hectorie, polyethylene, acrylate copolymers etc | 0% - 50% |
| Pigments e.g. Iron oxide | 5% - 15% |
| Preservatives | 0.1% - 0.5% |
| Other additives Triethanolamine, Ammonia, Disodium EDTA | 0.1% - 1% |

Depending on the amount of water, the composition may be hydrophilic or lipophilic. The powder concentrate is uniformly mixed with the other powders and dispersed either in the hydrophilic or lipophilic phase using high speed stirring or milling depending on the viscosity of the composition..

### Example 13

In this example for colour cosmetic compositions, the ambiphilic powder concentrate is dispersed with the pigments and incorporated with the hydrophilic or lipophilic ingredients, unless otherwise stated.
1. Foundations: 1-10 wt % Ambiphilic porous powder concentrate from Ex 1 dispersed in the aqueous phase of the formulation, to act as a hydrator and sebum absorber in the final product.
   Types of foundations: O/W and W/O
2. Foundations: 1 % to 10 wt % Ambiphilic porous powder concentrate from Ex 1 (omitting the polymer) with hydrophilic or lipophilic components loaded on so the product is still a powder, to act as a hydrator/moisturiser and sebum absorber in the final product
   Types of foundations: Powder and loose-powder, and anhydrous foundations ("cream-powder and liquid-powder").
3. Blushers: 1-10 wt% Ambiphilic porous powder concentrate from Ex 1 (without the polymer) with hydrophilic or lipophilic components loaded on so the product is still a powder, to acts as a hydrator/moisturiser and sebum absorber in the final blusher product
   Types of blushers: powder blush and anhydrous blush (waxed-based)
4. Lipsticks and lip pencils: 1-10 wt% Ambiphilic porous powder concentrate from Ex 1 (omitting the polymer) with hydrophilic components loaded on to act as a hydrating reservoir in the lipstick.
5. Mascara: 1-10 wt% Ambiphilic porous powder concentrate from Ex 1 (omitting the polymer) with hydrophilic or liquid active components loaded on so the product is still a powder, to act as lash conditioning, hydrating, moisturising component in the final mascara product.
   Types of mascaras: anhydrous, W/O and O/W.
6. Eye shadow powders and sticks: 1-10 wt% Ambiphilic porous powder concentrate from Ex 1 (omitting the polymer) with hydrophilic or liquid active components (e.g. anti-ageing ingredients) loaded on so the product is still a powder, to act as a conditioning/moisturising component for the eyes.

### Example 14

1 wt% to 10 wt% ambiphilic powder concentrate as in Ex 1 incorporated into aqueous-alcohol gel base comprising a metallic soap for preparing antiperspirant/deodorising solid or stick.

The compositions described are capable of forming external reservoirs comprising a polymeric matrix for sebum regulation by absorbing sebum in exchange for beneficial oils and facilitate transfer of hydrating components to the skin. The compositions may be tonics, lotions, creams, gels, pastes, powders compressed or loose, oil in water, water in oil systems, and lip sticks. There is also described a method of preparing said lotions, creams, etc containing an ambiphilic powder mixture or complex comprising porous particles, a lipophilic component, hydrophilic attractant, hydrophilic polymer, optionally biologically active compound and excipients.

## Claims

1. An ambiphilic powder mixture for use in topical compositions which consists of
a) 25 wt % to 75 wt % water insoluble synthetic porous agglomerates of inorganic compounds with specific surface area greater than 40 m²/g made from individual particles having a mean diameter from 0.01 µm to 0.1 µm, said porous agglomerates having the capacity to absorb from 1- 5 times their weight of a liquid, blended with
b) 5 wt % to 25 wt %of at least one lipophilic compound complexed with said porous agglomerates to render the particles lipophilic
c) 1 wt % to 25 wt %of at least one hydrophilic component
d) 0.5 wt % to 10 wt % of at least one physiologically acceptable surfactant
e) less than 10 wt % water or bound moisture.

2. The ambiphilic powder mixture of claim 1 further including
f) 1 wt% to 10 wt% of at least one biologically active compound and excipients.

3. The ambiphilic powder mixture according to claim 1 or 2, wherein the water insoluble synthetic porous agglomerates of inorganic compounds with specific surface area greater than 40 m²/g consist of a synthetic porous material selected from the group consisting of amorphous Al2O3•MgO•2SiO2•nH2O, CaHPO4•mH2O, CaSiO4, Ca3SiO5 and dibasic anhydrous calcium phosphate.

4. A lotion, gel, cream or paste like skin-care composition obtainable by homogeneously mixing and dispersing 1 wt % to 10 wt % of the ambiphilic powder composition of any one of claims 1 - 3 in a suitable base carrier.

5. A skin-care composition prepared in accordance with claim 4 for adsorbing oils and hydrating skin, scalp and hair.

6. A skin-care composition to absorb sebum and hydrate skin comprising an ambiphilic dispersed system according to claim 1 consisting of
a) water insoluble synthetic porous agglomerates of organic and/or inorganic compounds with specific surface area greater than 40 m²/g selected from the group consisting of amorphous calcium silicate, silica, porous silicone dioxide, silicic acid, dibasic anhydrous calcium phosphate, polystyrene beads or microsponges,
b) at least one lipophilic component selected from the group consisting of low viscosity grades of ethyl cellulose, natural gums such as Dammar gum, black cumin seed oil, pumpkin seed oil, wheat germ oil, tocopherols, volatile oils, volatile silicones, hydrogenated poly isobutane,
c) at least one hydro-active compound dissolved with a swollen hydrophilic polymer, selected from the group consisting of water dispersible cellulose esters and ethers, natural gums and hydrocolloids such as gelatin, collagen, proteins, amino acids, peptides, polypeptides, carbomers, carboxy vinyl co-polymers, sodium acrylates, Dammar gum and low viscosity grades of ethyl cellulose such as N7, N4 0r N3 (Dow Chemical or Hercules Inc) or equivalent,
d) at least one amphiphilic or amphipathic surfactant, mixed with
e) 10 wt % to 99 wt % hydrophilic, lipophilic or particulate carrier, that provides a reservoir for lubricating and hydrating the skin.

7. Use of a skin-care composition according to claim 6 to absorb sebum and hydrate skin.

## Patentansprüche

1. Ambiphile Pulvermischung zur Verwendung in Zusammensetzungen zur lokalen Anwendung, bestehend aus
a) 25 bis 75 Gewichtsprozent wasserunlöslicher synthetischer poröser Agglomerate anorganischer Verbindungen mit einem spezifischen Oberflächenwert von mehr als 40 m²/g, welche aus einzelnen Partikeln hergestellt sind, welche einen mittleren Durchmesser von 0,01 µm bis 0,1 µm aufweisen, wobei die porösen Agglomerate die Kapazität aufweisen, eine Flüssigkeit in einer Menge des Ein- bis Fünffachen von deren Gewicht zu absorbieren, wobei diese versetzt sind mit
b) 5 bis 25 Gewichtsprozent mindestens einer lipophilen Verbindung, welche mit den porösen Agglomeraten zu Komplexen verbunden ist, um die lipophilen Partikel zu liefern,
c) 1 bis 25 Gewichtsprozent mindestens einer hydrophilen Komponente,
d) 0,5 bis 10 Gewichtsprozent mindestens eines physiologisch annehmbaren Benetzungsmittels und
e) weniger als 10 Gewichtsprozent Wasser bzw. gebundener Feuchtigkeit.

2. Ambiphile Pulvermischung nach Anspruch 1, ferner umfassend
f) 1 bis 10 Gewichtsprozent mindestens einer biologisch aktiven Verbindung und pharmazeutischer Hilfsstoffe.

3. Ambiphile Pulvermischung nach Anspruch 1 oder 2, wobei die wasserunlöslichen synthetischen porösen Agglomerate anorganischer Verbindungen mit einem spezifischen Oberflächenwert von mehr als 40 m²/g aus einem synthetischen porösen Material bestehen, welches aus der Gruppe ausgewählt ist, welche aus amorphem Al₂O₃•MgO•2SiO₂•nH₂O, CaHPO₄•mH₂O, CaSiO₄, Ca₃SiO₅ und zweibasigem wasserfreiem Kalziumphosphat besteht.

4. Lotion-, gel-, creme- oder pastenartige Hautpflegezusammensetzung, welche durch gleichmäßiges Mischen und Dispergieren von 1 bis 10 Gewichtsprozent der ambiphilen Pulvermischung nach einem der Ansprüche 1 bis 3 in einem geeigneten Basis-Trägermaterial herstellbar ist.

5. Hautpflegezusammensetzung, welche nach Anspruch 4 zubereitet ist, zum Adsorbieren von Ölen und zum Hydratisieren von Haut, Kopfhaut und Haar.

6. Hautpflegezusammensetzung zum Absorbieren von Talg und zum Hydratisieren der Haut, umfassend ein ambiphiles dispergiertes System nach Anspruch 1, bestehend aus
a) wasserunlöslichen synthetischen porösen Agglomeraten organischer und/oder anorganischer Verbindungen mit einem spezifischen Oberflächenwert von mehr als 40 m²/g, welche aus der Gruppe ausgewählt sind, welche aus amorphem Kalziumsilikat, Silikamaterial, porösem Siliziumdioxid, Kieselsäure, zweibasigem wasserfreiem Kalziumphosphat, Polystyrolperlen oder - mikroschwämmen besteht,
b) mindestens einer lipophilen Komponente, welche aus der Gruppe ausgewählt ist, welche aus niederviskosen Typen von Ethylzellulose, Naturkautschuken, wie etwa Dammarharz, schwarzem Kreuzkümmelsamenöl, Kürbiskernöl, Weizenkeimöl, Tocopherolen, ätherischen Ölen, flüchtigen Silikonen und hydriertem Polyisobutan besteht,
c) mindestens einer hydroaktiven Verbindung, welche mit einem gequollenen hydrophilen Polymer gelöst ist, welche aus der Gruppe ausgewählt ist, welche aus in Wasser dispersionsfähigen Zelluloseestern und -ethern, Naturkautschuken und Hydrokolloiden, wie etwa Gelatine, Kollagen, Proteinen, Aminosäuren, Peptiden, Polypeptiden, Karbomeren, Karboxy-Vinyl-Kopolymeren, Natriumacrylaten, Dammerharz und niederviskosen Typen von Ethylzellulose, wie etwa N7, N4 oder N3 (Dow Chemical bzw. Hercules Inc.) oder äquivalenten Materialien besteht,
d) mindestens einem ambiphilen bzw. amphipatischen Benetzungsmittel, gemischt mit
e) 10 bis 99 Gewichtsprozent eines hydrophilen, lipophilen oder partikelförmigen Trägermaterials, welches ein Reservoir zum Ölen und Hydratisieren der Haut liefert.

7. Verwendung einer Hautpflegezusammensetzung nach Anspruch 6 zum Absorbieren von Talg und zum Hydratisieren der Haut.

## Revendications

1. Mélange de poudre ambiphile pour l'utilisation dans les compositions topiques, lequel consiste en
a) 25 % du poids à 75 % du poids d'agglomérats synthétiques poreux insolubles dans l'eau de composés inorganiques avec une surface spécifique supérieure à 40 m²/g et composés de particules individuelles possédant un diamètre moyen de 0,01 µm à 0,1 µm, lesdits agglomérats poreux possédant la capacité d'adsorber une quantité de liquide de 1 à 5 fois leur poids, mélangés à
b) 5 % du poids à 25 % du poids d'au moins un composé lipophile, complexé avec lesdits agglomérats poreux pour rendre les particules lipophiles,
c) 1 % du poids à 25 % du poids d'au moins un composant hydrophile,
d) 0, 5 % du poids à 10 % du poids d'au moins un agent tensioactif physiologiquement acceptable,
e) moins de 10 % d'eau ou d'eau liée.

2. Mélange de poudre ambiphile selon la revendication 1, comportant en outre
f) 1 % du poids à 10 % du poids d'au moins un composé biologiquement actif et des excipients

3. Mélange de poudre ambiphile selon l'une quelconque des revendications 1 ou 2, dans laquelle les agglomérats synthétiques poreux insolubles dans l'eau de composés inorganiques avec une surface spécifique supérieure à 40 m²/g consistent en une matière synthétique poreuse sélectionnée à partir du groupe consistant en le Al2O3-MgO-2SiO2-nH2O amorphe, le CaHPO4-mH20, le CaSiO4, le Ca3SiO5 et le phosphate de calcium dibasique anhydre.

4. Composition de soin pour la peau de type lotion, gel, crème ou pâte obtenue en mélangeant et en dispersant de manière homogène 1 % du poids à 10 % du poids de la composition de poudre ambiphile selon l'une quelconque des revendications 1 à 3 dans un support de base approprié.

5. Composition de soin pour la peau préparée en accord avec la revendication 4 pour adsorber les huiles et hydrater la peau, le cuir chevelu et les cheveux.

6. Composition de soin pour la peau pour absorber le sébum et hydrater la peau, comportant un système ambiphile dispersé selon la revendication 1, consistant en
a) des agglomérats synthétiques poreux insolubles dans l'eau de composés organiques et/ou inorganiques avec une surface spécifique supérieure à 40 m²/g, sélectionnés du groupe consistant en le silicate de calcium amorphe, le silice, le dioxyde de silicone poreux, l'acide silicique, le phosphate de calcium dibasique anhydre, des billes de polystyrène ou des microéponges,
b) au moins un composant lipophile sélectionné du groupe consistant en des types à basse viscosité d'éthylcellulose, de gommes naturelles telles que la résine dammar, d'huile de graines de cumin noir, d'huile de graines de citrouille, d'huile de germes de blé, de tocophérols, d'huiles volatiles, de silicones volatiles, de polyisobutane hydrogéné,
c) au moins un composé hydroactif dissout dans un polymère hydrophile expansé, sélectionné du groupe consistant en des esters cellulosiques hydrodispersables et des éthers, des gommes naturelles et des hydrocolloïdes tels que la gélatine, le collagène, les protéines, les aminoacides, les peptides, les polypeptides, les carbomères, les carboxy-copolymères de vinyle, les acrylates de sodium, la résine dammar et des types à basse viscosité d'éthylcellulose tels que le N7, le N4 ou le N3 (Dow Chemical ou Hercules Inc) ou des matières équivalentes,
d) au moins un agent tensioactif amphiphile ou amphipathique, mélangé avec
e) 10 % du poids à 99 % du poids d'un support hydrophile, lipophile ou particulaire,
lequel fournit un réservoir pour graisser et hydrater la peau.

7. Utilisation d'une composition de soin pour la peau selon la revendication 6 pour absorber le sébum et hydrater la peau.
